## Europäisches Patentamt
## European Patent Office
## Office européen des brevets

(19)

(11) Numéro de publication: **0 099 305 B1**

(12) # FASCICULE DE BREVET EUROPEEN

(45) Date de publication du fascicule du brevet:
**07.01.88**

(51) Int. Cl.⁴: **A 61 L 9/00**, A 61 G 17/00

(21) Numéro de dépôt: **83401460.7**

(22) Date de dépôt: **13.07.83**

(54) **Appareil de désinfection des éffluents gazeux dans les cellules mortuaires étanches.**

(30) Priorité: **16.07.82 FR 8212449**

(43) Date de publication de la demande:
**25.01.84 Bulletin 84/4**

(45) Mention de la délivrance du brevet:
**07.01.88 Bulletin 88/1**

(84) Etats contractants désignés:
**BE CH DE IT LI NL**

(56) Documents cités:
**DE - B - 1 196 320**
**FR - A - 2 455 917**
**US - A - 4 162 289**

(73) Titulaire: **DETERLUB S.a.r.l., 1325 Route de Vallauris,
F-06250 Mougins (FR)**
Titulaire: **Augias, Francis, La Giavis La Moutonne,
F-83260 La Crau (FR)**

(72) Inventeur: **Augias, Francis, la Giavis La Moutonne,
F-83260 La Crau (FR)**
Inventeur: **Roard, Dany, 1325 Route de Vallauris,
F-06250 Mougins (FR)**

(74) Mandataire: **Ores, Irène et al, CABINET ORES 6, Avenue
de Messine, F-75008 Paris (FR)**

## Description

La présente invention est relative à un appareil (1) de désinfection des effluents gazeux d'une cellule mortuaire étanche tel que défini ci-dessous comprenant une enceinte close de désinfection, un conduit d'arrivée d'air contenant les effluents gazeux de décomposition pourvu d'un orifice de sortie de l'air à traiter, un milieu liquide de désinfection de cet air et une tubulure d'évacuation pourvue d'un orifice d'entrée de l'air traité.

Les sépultures en caveaux, qui sont généralement des concessions, permettent une destruction plus rapide des corps à condition d'assurer une ventilation permanente. C'est pourquoi certains techniciens préconisent leur généralisation sous une forme préfabriquée. Cela apporterait de nombreux avantages: réduction du travail des fossoyeurs, moindres frais, meilleure utilisation de la superficie des cimetières, destruction plus rapide et plus complète des corps, salubrité meilleure en cas d'exhumation. Il s'agirait de réaliser en sous-sol, des sépultures identiques à celles en enfeux. Les enfeux très répandus en Espagne, en Italie et dans certaines régions de France, sont constitués par des blocs de cases aménagées au-dessus du sol. Leurs parois sont faites de matériaux poreux qui facilitent la libre circulation de l'air et qui évitent une anaérobiose homogène des corps. Naturellement, la case ne doit pas permettre l'accès des insectes. Lorsque ces conditions sont réalisées, la destruction des corps est réalisée en trois à cinq ans, sans odeur décelable et sans risque de pollution des nappes souterraines.

Toutefois, dans un très grand nombre de cas, les sépultures sont encore constituées de cellules étanches. Les phénomènes de décomposition des cadavres en cellules étanches rendent difficiles les interventions nécessaires d'exhumation pour une nouvelle inhumation, imposées par la législation sur les sépultures ou le code des communes.

On connaît déjà, par exemple par US-A-4 162 289, un filtre de désinfection des effluents gazeux d'une cellule mortuaire étanche qui comprend une cartouche résistant à la corrosion et enfermant différentes couches de matériaux pour l'élimination des gaz délétères et/ou malodorants qui résultent de la décomposition des cadavres. Un tel filtre, qui est prévu pour être installé de manière fixe, ne convient pas pour équiper des sépultures dans lesquelles doivent avoir lieu des inhumations successives.

On connaît également, par FR-A-2 455 917, un dispositif d'épuration et de désinfection des gaz cadavériques destiné à équiper un cercueil. Le dispositif, qui comprend un liquide d'épuration et un moyen de flotteur n'est pas prévu, à proprement parler, pour des sépultures en caveau. Il en est de même du dispositif d'absorption pour cercueil décrit dans DE-B-1 196 320.

A partir de cet état de la technique, la présente invention a pour but de pourvoir les cellules mortuaires étanches d'un appareil de désinfection des effluents gazeux, propre à éviter les risques de pollution de l'environnement qui soit approprié pour équiper des caveaux dans lesquels ont lieu des inhumations successives.

Pour atteindre ce but, l'appareil de désinfection des effluents gazeux d'une cellule mortuaire étanche est caractérisé en ce que le conduit d'arrivée de l'air à traiter traverse la paroi supérieure de l'enceinte close, l'orifice de sortie de l'air à traiter étant situé à la partie inférieure de ladite enceinte, l'orifice d'entrée de l'air traité dans la tubulure d'évacuation étant situé au voisinage de la paroi supérieure de l'enceinte close, tandis que ladite tubulure d'évacuation de l'air traité comporte à son extrémité non reliée à l'enceinte close un dispositif adaptateur permettant le remplissage de l'enceinte close par un liquide désinfectant, le milieu liquide de désinfection étant interposé en-tre l'orifice de sortie de l'air à traiter et l'orifice d'entrée de l'air traité, avec un garnissage d'un granulat disposé entre l'orifice de sortie de l'air à traiter et l'orifice d'entrée de l'air traité, ledit garnissage étant séparé de ces orifices et étant supporté par un diffuseur situé au-dessus de l'orifice d'entrée de l'air à traiter de sorte qu'il est propre à forcer la division des bulles d'air à traiter afin d'assurer un contact optimal dudit air avec le liquide de désinfection.

D'autres caractéristiques de l'appareil sont indiquées dans les sous-revendications.

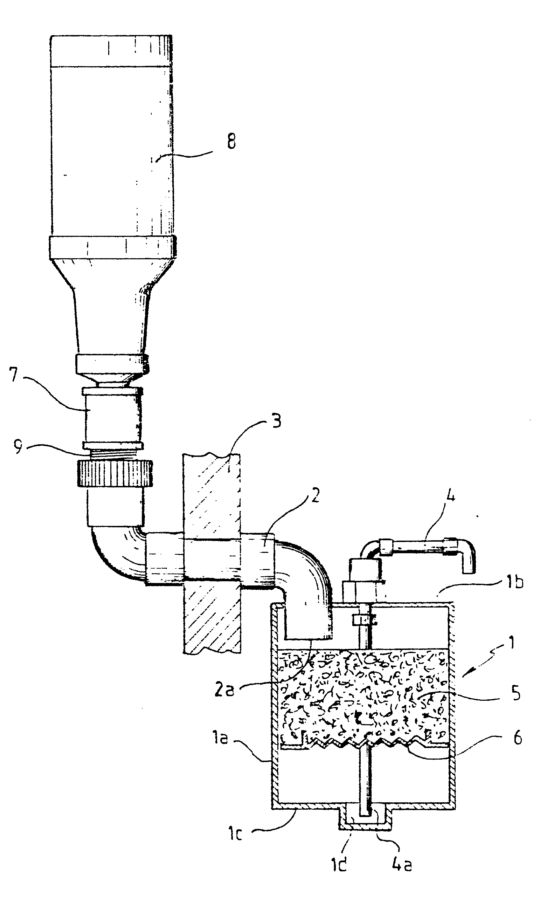

L'invention sera mieux comprise à l'aide du complément de description qui va suivre, qui se réfère au dessin annexé dans lequel la Figure unique est une vue d'ensemble de l'appareil, l'enceinte close étant représentée en coupe frontale.

L'appareil (1) comprend une enceinte close de désinfection 1, de préférence cylindrique, avec une paroi latérale 1a, une paroi d'extrémité supérieure 1b et une paroi d'extrémité inférieure 1c.

L'enceinte est disposée à l'intérieur d'une cellule mortuaire étanche, reliée à l'extérieur de la-dite cellule par la tubulure 2 dite tubulure d'évacuation de l'air traité, laquelle traverse la paroi, référencée 2, de la cellule mortuaire étanche.

La tubulure d'évacuation de l'air traité 2 présente un orifice d'évacuation de l'air traité 2a qui débouche à la partie supérieure de l'enceinte close de désinfection 1.

Une conduite d'arrivée 4 de l'air contenant les effluents gazeux de décomposition traverse la paroi supérieure 1b de l'enceinte close 1 et débouche à la partie inférieure de ladite enceinte 1.

Entre l'orifice de sortie 4a de la conduite 4 et l'orifice 2a de la tubulure d'évacuation 2, il est prévu un garnissage interne 5 d'un granulat, de granulométrie voisine de celui obtenu entre les tamis 34 et 35 de la norme AFNOR (mailles respectives 2 et 2,5 mm de côté), par exemple, lequel granulat est supporté par un diffuseur 6 en forme de plateau transversal.

A l'extrémité de la tubulure d'évacuation de l'air traité 2 peut être monté un adaptateur 7 par simple vissage, permettant le montage d'une cartouche 8 de liquide désinfectant.

Le liquide désinfectant peut être un mélange ammonium quaternaire-glycols-butylglycol, et comporter éventuellement des substances colorantes et/ou aromatiques.

La conduite d'arrivée 4 est de préférence en cuivre, tandis que l'enceinte close de désinfection 1, le diffuseur 6 et la tubulure d'évacuation 2 peuvent être en matière plastique, de préférence en PVC.

A titre d'exemple de l'objet de l'invention, l'enceinte close de désinfection peut être en cylindre de diamètre 130 mm et de hauteur 140 mm, le diffuseur étant placé à 40 mm environ de la paroi inférieure 1c, et le garnissage interne de granulat pouvant atteindre environ 105 mm à compter de cette paroi inférieure 1c.

L'appareil fonctionne de la manière suivante:

En premier lieu, l'appareil est scellé dans la cellule mortuaire étanche au moyen de la tubulure d'évacuation 2 qui traverse un orifice d'un paroi 3 de ladite cellule mortuaire.

On procède au remplissage de l'enceinte de désinfection.

Le premier remplissage s'effectue sans adaptateur 7, pour permettre l'évacuation de l'air. Le système est conçu pour les remplissages suivants effectués au moyen de l'adaptateur 7 et de la cartouche 8 permettant l'évacuation par le bas de l'ancien liquide par le conduit 4 en écoulement laminaire. Cette disposition permet la substitution du nouveau liquide à l'ancien, sans mélange.

A cette fin, la paroi inférieure 1c de l'enceinte close présente une cuvette 1d en regard de l'orifice de sortie 4a du conduit 4.

Le liquide désinfectant évacué par le conduit 4, aux capacités désinfectantes encore très élevées, s'écoule sur des panneaux d'absorption des effluents liquides placés au bas de la cellule mortuaire, de manière à renforcer les propriétés désinfectantes de l'ensemble.

Toute surpression de l'air ambiant de la cellule mortuaire supérieure à 7 g/cm² est évacuée au travers de l'appareil. L'air comportant les effluents gazeux de la décomposition parcourt le conduit d'arrivée 4 pour atteindre la partie inférieure de l'appareil, puis remonte jusqu'au diffuseur 6. Le garnissage de granulat 5 oblige les bulles à se diviser finement pour assurer un contact optimal avec le liquide désinfectant qui occupe tout ou partie de l'espace intérieur de l'enceinte 1. En pratique, il est nécessaire de remplir l'enceinte 1 de telle manière que la longueur de traversée de liquide désinfectant soit égale ou supérieure à 55 mm.

L'air désinfecté est évacué par la tubulure d'évacuation 2.

L'appareil permet un nouveau remplissage de liquide désinfectant pour une nouvelle inhumation. Pour cela, l'orifice de sortie de la tubulure d'évacuation 2 de l'air traité se termine par un embout 9 fileté, vertical, dirigé vers le haut, apte à recevoir un adaptateur 7 pour le remplissage au moyen d'une cartouche 8 de liquide désinfectant. Cet adaptateur 7 est enlevé après l'opération de remplissage et est remplacé par un chapeau d'évacuation, non représenté.

Le remplissage est effectué avec un liquide désinfectant (bactéricide), désodorisant spécial à très faible volatilité, hydrosoluble. La quantité contenue dans une cartouche est suffisante pour dépasser le niveau du lit de granulat.

L'air qui entre dans l'appareil avec un degré de pollution très élevé, sort de l'appareil avec une parfaite propreté bactérienne.

Cet appareil est étudié, avec sa cartouche de liquide désinfectant, pour suffire au traitement d'une inhumation. Il convient pour toute nouvelle inhumation dans la cellule mortuaire d'effectuer le renouvellement du liquide désinfectant.

## Revendications

1. Appareil de désinfection des effluents gazeux d'une cellule mortuaire étanche, comprenant une enceinte close de désinfection (1), un conduit d'arrivée d'air (4) contenant les effluents gazeux de décomposition pourvu d'un orifice de sortie (2a) de l'air à traiter, un milieu liquide de désinfection de cet air et une tubulure d'évacuation (2) pourvue d'un orifice d'entrée (2a) de l'air traité, caractérisé en ce que:
– le conduit d'arrivée (4) de l'air à traiter traverse la paroi supérieure (1b) de l'enceinte close (1), l'orifice de sortie (4a) de l'air à traiter étant situé à la partie inférieure de ladite enceinte,
– l'orifice d'entrée (2a) de l'air traité dans la tubulure d'évacuation (2) est situé au voisinage de la paroi supérieure (1b) de l'enceinte close (1), tandis que ladite tubulure d'évacuation (2) de l'air traité comporte à son extrémité non reliée à l'enceinte close un dispositif adaptateur (7) permettant le remplissage de l'enceinte close (1) par un liquide désinfectant,
– le milieu liquide de désinfection est interposé entre l'orifice de sortie (4a) de l'air à traiter et l'orifice d'entrée (2a) de l'air traité, et
– un garnissage d'un granulat (5) est disposé entre l'orifice de sortie (4a) de l'air à traiter et l'orifice d'entrée (2a) de l'air traité, tout en étant séparé de ces orifices, et est supporté par un diffuseur (6) situé au-dessus de l'orifice d'entrée (2a) de l'air à traiter, lequel granulat est propre à forcer la division des bulles d'air à traiter afin d'assurer un contact optimal de cet air avec ledit liquide de désinfection.

2. Appareil selon la revendication 1, caractérisé en ce que ledit dispositif (7) est adapté pour recevoir à chaque inhumation, notamment par vissage ou par un autre moyen connu équivalent, une cartouche (8) de liquide désinfectant pour effectuer le remplissage de l'enceinte close (1) de désinfection lors de la première inhumation ou le renouvellement du liquide de désinfection à chaque nouvelle inhumation.

3. Appareil selon la revendication 1, caractérisé en ce que la garniture interne de granulat (5) présente une granulométrie de l'ordre de celui obtenu entre les tamis 34 et 35 de la norme AFNOR (mailles respectives 2 et 2,5 mm de côté).

4. Appareil selon l'une quelconque des revendications 1 à 3, caractérisé en ce que ledit liquide désinfectant est un liquide hydrosoluble et à très faible volatilité.

5. Appareil selon la revendication 4, caractérisé en ce que le liquide désinfectant est un mélange ammonium quaternaire-glycols-butylglycol, comprenant éventuellement des substances colorantes et/ou aromatiques.

6. Appareil selon l'une quelconque des revendications 1 à 5, caractérisé en ce que le conduit d'entrée (4) d'air contenant les effluents gazeux de décomposition est en cuivre.

7. Appareil selon l'une quelconque des revendications 1 à 6, caractérisé en ce que l'enceinte close (1) de désinfection et la tubulure d'évacuation (2) de l'air traité, sont en matière plastique, de préférence en polychlorure de vinyle.

**Claims**

1. Apparatus for disinfecting the gaseous effluents from a sealed mortuary cell, comprising a closed disinfection enclosure (1), an air intake duct (4) containing the gaseous decomposition effluents and having an outlet orifice (2a) for the air to be treated, a liquid medium for disinfecting this air and a discharge pipe (2) with an inlet orifice (2a) for the treated air, characterized in that:
— the intake duct (4) for the air to be treated passes through the upper wall (1b) of the closed enclosure (1), the outlet orifice (4a) for the air to be treated being situated at the lower part of said enclosure,
— the intake orifice (2a) for the treated air in the discharge pipe (2) is situated in the vicinity of the upper wall (1b) of the closed enclosure (1), whereas, said discharge pipe (2) for the treated air comprises at its end not connected to the closed enclosure an adaptor device (7) for filling the closed enclosure (1) with a disinfectant liquid,
— the liquid disinfection medium is interposed between the outlet orifice (4a) for the air to be treated and the intake orifice (2a) for the treated air, and
— a granulate packing (5) is disposed between the outlet orifice (4a) for the air to be treated and the intake orifice (2a) for the treated air, while being separated from these orifices, and is supported by a diffuser (6) situated above the intake orifice (2a) for the air to be treated, which granulate is capable of forcing the division of the air bubbles to be treated so as to ensure optimum contact of this air with said disinfection liquid.

2. Apparatus according to claim 1, characterized in that said device (7) is adapted for receiving at each internment, more particularly by screwing or by any other equivalent known means, a cartridge (8) of disinfectant liquid for filling the closed disinfection enclosure (1) during the first internment or for renewing the disinfection liquid at each new internment.

3. Apparatus according to claim 1, characterized in that the internal granulate packing (5) has a grain size of the order of that obtained between screens 34 and 35 of the AFNOR standard (respective mesh of 2 and 2.5 m$^2$).

4. Apparatus according to any one of claims 1 to 3, characterized in that said disinfectant liquid is a hydrosoluble liquid with very low volatility.

5. Apparatus according to claim 4, characterized in that the disinfectant liquid is a quaternary ammonium-glycols-butylglycol mixture, possibly including dye and/or aromatic substances.

6. Apparatus according to any one of claims 1 to 5, characterized in that the intake duct (4) for the air containing the gaseous decomposition effluents is made from copper.

7. Apparatus according to any one of claims 1 to 6, characterized in that the closed disinfection enclosure (1) and the pipe (2) for discharging the treated air are made from a plastic material, preferably from polyvinylchloride.

**Patentansprüche**

1. Vorrichtung zur Desinfektion von in luftdichten Leichenkammern entstehenden Gasen mit einem geschlossenen Desinfektionsraum (1), in den eine Zuführungsleitung (4) für die, die Verwesungsgase enthaltende Luft mündet, ferner eine Ausgangsmündung (2a) für die desinfizierte Luft, eine Füllung mit einer Flüssigkeit zur Desinfektion der zugeführten Luft und ein Abführungsrohr (2) mit Eingangsmündung (2a) für die desinfizierte Luft, aufweist, gekennzeichnet durch folgende Merkmale:
— die Zuführungsleitung (4) für die zu desinfizierende Luft durchdringt die obere Wandung (1b) des geschlossenen Desinfektionsraums (1), wobei die Ausgangsmündung (4a) der Leitung für die zu desinfizierende Luft im unteren Bereich des geschlossenen Desinfektionsraums liegt,
— die Eingangsmündung (2a) für die desinfizierte Luft des Abführungsrohrs (2) ist im Bereich der oberen Wand (1b) des geschlossenen Raums (1) angeordnet, wobei das Abführrohr (2), für die desinfizierte Luft an seinem nicht mit dem geschlossenen Raum verbundenen Ende einen Adapter (7) aufweist, der ein Nachfüllen des geschlossenen Raums (1) mit flüssigem Desinfektionsmittel gestattet,
— das flüssige Desinfektionsmittel ist zwischen der Ausgangsmündung (4a) der Leitung für die zu desinfizierende Luft und der Eingangsmündung (2a) für die desinfizierte Luft angeordnet und
— eine Granulatanordnung (5) ist zwischen der Ausgangsmündung (4a) für die zu desinfizierende Luft und die Eingangsmündung (2a) für die desinfizierte Luft in jeweiligem Abstand von den Mündungen angeordnet und wird von einer die Diffusion bewirkenden Unterlage (6) getragen, die unterhalb der Eingangsmündung (2a) für die desinfizierte Luft angeordnet ist, wobei das Granulat geeignet ist, die Zerteilung der Luftbläschen zu bewirken, um einen optimalen Kontakt der Luft mit dem flüssigen Desinfektionsmittel zu bewirken.

2. Vorrichtung nach Anspruch 1, dadurch gekennzeichnet, dass der Adapter (7) in jeglicher Weise vorzugsweise durch Verschraubung oder ein anderes äquivalentes Mittel mit einem Behälter (8) für flüssiges Desinfektionsmittel in Verbindung steht, zum Zwecke der Bewirkung der Wiederauffüllung des geschlossenen Raums (1) mit Desinfektionsmittel nach der ersten Desinfektion oder zur Ergänzung mit flüssigem Desinfektionsmittel zu jeder neuen Desinfektion.

3. Vorrichtung nach Anspruch 1–2, dadurch gekennzeichnet, dass die Granulatanordnung (5) eine solche Granulometrie aufweist, die durch Siebung mit Sieben Nr. 34 und Nr. 35 der Norm AFNOR d.h. mit Maschenweiten von 2 mm bzw. 2,5 mm erhalten wurden.

4. Vorrichtung nach Ansprüchen 1–3, dadurch gekennzeichnet, dass das flüssige Desinfektionsmittel aus einer wasserlöslichen Flüssigkeit mit sehr geringer Flüchtigkeit besteht.

5. Vorrichtung nach Anspruch 4, dadurch gekennzeichnet, dass das flüssige Desinfektionsmittel aus einem Gemisch von quarternärem Ammoniumglykol-butylglykol besteht, das gegebenenfalls gefärbte Substanzen und/oder Aromasubstanzen enthält.

6. Vorrichtung nach Ansprüchen 1 bis 5, dadurch gekennzeichnet, dass die Zuführleitung (4) für die Verwesungsgase enthaltende Luft aus Kupfer besteht.

7. Vorrichtung nach Ansprüchen 1 bis 6, dadurch gekennzeichnet, dass der geschlossene Desinfektionsraum (1) und das Abführungsrohr (2) für die desinfizierte Luft aus Kunststoff, vorzugsweise aus Polyvinylchlorid besteht.